# EUROPEAN PATENT APPLICATION

(11) **EP 2 520 256 A1**
(43) Date of publication of application: **07.11.2012**
(21) Application number: 12176564.8
(22) Date of filing: 03.11.2008
(51) Int. Cl.: A61F 2/84

(54) **Graft fenestration fabric**

(30) Priority: 01.11.2007 GB 0721459
(62) Divisional of application: 08844291.8
(71) Applicant: VASCUTEK LIMITED, Renfrewshire, Scotland PA4 9RR (GB)
(72) Inventor: Berg, Patrick, 8261 Mamer (LU)
(74) Representative: Ouzman, Beverley Nicola Claire

(57) **Abstract**

The present invention provides a graft fabric adapted for fenestration, wherein an area of the graft is reinforced by a mesh comprising an arrangement of strands, characterised in that the strands forming the mesh are woven or knitted into the fabric itself during construction. During fenestration, the edges of the tear are limited to the open area of the mesh and propagation of tears in the fabric is resisted.

## Description

The present invention relates to apparatus for location of a site for fenestration of a graft in situ and to a method of locating a site for fenestration of a graft in situ.

Aortic aneurysm is characterised by the dilation or ballooning out of part of the wall of the aorta, the artery through which blood flows from the heart to the body. The majority of aortic aneurysms cause little or no symptoms, and small aneurysms can be controlled with beta blockers to reduce blood pressure. However, larger aneurysms pose significant danger to the patient, as rupture of an aneurysm is frequently fatal. An aneurysm may be caused by genetic conditions, e.g. Marfan syndrome, or by other diseases, and forms when the wall of the artery becomes weakened, often due to the build up of plaque (arteriosclerosis). High blood pressure can also increase the likelihood of aneurysm development. Rupture of an aortic aneurysm gives only a 20% chance of survival, so there is a significant emphasis placed on early diagnosis and treatment. In most cases the abdominal aorta, supplying blood to the abdomen, the pelvis, and the legs, is affected (abdominal aorta aneurysm; AAA), but the thoracic aorta can also be susceptible to aneurysm (thoracic aorta aneurysm; TAA).

Aneurysms of the abdominal and thoracic aorta are thus a major cause of mortality, especially in western societies. With an increasingly ageing population, the incidence of aneurysm, particularly abdominal aortic aneurysm, continues to rise.

Provided that an aneurysm is diagnosed prior to rupture, surgical treatment to repair the affected vessel wall is effective. Surgical treatment of aneurysm involves the replacement or reinforcement of the aneurismal section of aorta with a synthetic graft or prosthesis under general anaesthesia allowing the patient's abdomen or thorax to be opened (see Parodi et al., Annals of Vascular Surgery (1991) 5:491-499). The patient will then have a normal life expectancy.

Surgical repair of aneurysm is however a major and invasive undertaking and there has been much effort in developing less invasive methods. Currently, aneurysm repair generally involves the delivery by catheter of a fabric or ePTFE graft which is retained at the required location by deployment of metallic stent elements. The ability to deliver the graft/stent device by catheter reduces the surgical intervention to a small cut-down to expose the femoral artery and, in suitable circumstances, the device can be deployed percutaneously. Catheter delivery is beneficial since the reduced invasive nature of the procedure allows utilisation of a local anaesthesic and leads to reduced mortality and morbidity, as well as decreased recovery time. For example, endovascular repair is typically used for repair of infra-renal abdominal aortic aneurysms where the graft is placed below the renal arteries. Many different types of devices useful for endovascular repair are now available, for example a resiliently engaging endovascular element described in US 6,635,080 (Vascutek) or a tubular fabric liner having a radially expandable supporting frame and a radiopaque marker element stitched to the liner as disclosed in US 6,203,568 (Medtronic).

However, whilst the endovascular repair of aneurysms is now accepted as the method of choice, the technique has significant limitations and is not suitable for all patients.

The difficulty of using a graft at or near an intersection of an artery to be repaired (such as the aorta) with branch vessels (for example the renal arteries, mesenteric artery, brachiocephalic artery, carotid arteries or left subclavian artery etc.) is well recognised. For such patients, endovascular repair is only possible using unique, individually designed and manufactured endografts having appropriate branch grafts or fenestrations which match the patient's individual anatomy. Whilst such grafts can be obtained commercially, for example the Zenith device of Cook, the meticulous design of such grafts is reliant on accurate pre-operative imaging data. The use of patient-specific designed grafts is expensive and requires significant pre-planning so that such grafts are not available in emergency situations. Moreover, the technical difficulties of aligning the fenestration to the branch vessel is significant, requiring precise axial and rotational control of the graft. No adjustments to the graft are possible during deployment, so that any errors in the initial diagnostic imaging or any changes in anatomy from the imaging stages cannot be rectified. An alternative approach requires the deliberate coverage of the branch vessel, which is clearly undesirable.

The desirability of conducting *in vivo* (in situ) fenestration of a graft has been recognised in the art. McWilliams et al., (J. Endovasc Ther (2004) 11:170-174) describe the fenestration of a thoracic graft by passing a guide wire down the branch artery to pierce the fabric of the graft and then expanding the hole formed by inflation of a balloon. However, whilst the technique described shows that in situ fenestration of a graft is possible, it requires percutaneous retrograde access to the branch vessel for correct location of the fenestration. Such access is possible for branches of the aortic arch but not for visceral vessels such as the renal or mesenteric arteries. Moreover, the requirement for retrograde access increases the complexity of the procedure.

An alternative approach is to puncture the graft fabric in an antegrade fashion (ie. from inside the graft lumen). Such a technique is described by Tse et al., (see J. Endovasc Ther (2007) 14:158-167). However Tse et al., experienced difficulties in siting the fenestration accurately from the graft lumen interior using intravenous ultrasound (IVUS), and multiple attempts at puncture were required. Moreover Tse et al., reported significant difficulties with the technique, including graft fracture.

McLachlan (WO 2007/082343) describes a method of creating fenestrations in situ through a body wall of a covered stent or endograft, using a specifically designed catheter.

Vortex (GB 2437058) describe apparatus for conducting in situ fenestration of a graft.

Taheri (US 5617878) describes the in situ fenestration of a graft, wherein location of the position of fenestration relies upon visualisation by intravenous ultrasound or angiogram. The difficulties of using intravenous ultrasound for such a purpose have, however, been described by Tse et al., above.

The use of magnets to join two guide wires inserted from different locations is described by Eton et al., (US 5,624,430), by Weiss (US 6,676,694) and by Lauterjung (US 6,264,662). Sharkaway et al., (US 7,241,300) refer to the use of magnetic coupling to aid anastomosis of grafts. None of the prior art suggests magnetic coupling to aid accurate location of a fenestration site on a graft.

The present invention provides an apparatus and method for accurate location of an in situ fenestration in a graft.

In one aspect, the present invention provides an apparatus for locating a site for in situ fenestration of a tubular graft, said apparatus comprising i) a first magnetic portion, and ii) a catheter having a second magnetic portion, wherein the first magnetic portion and the second magnetic portion are attracted to each other.

The first magnetic portion can be provided as part of a guide wire, or can be provided as part of a catheter. In the event that the first magnetic portion forms part of a catheter, the catheter is advantageously deployed over a guide wire.

Reference to magnetic portions (eg. the first and second magnetic portions) being "attracted to" each other as used herein refers to the magnetic force which attracts magnets of opposite polarity to each other and maintains the magnets in juxtaposition. The magnets, when held together by the attractive force, are then "docked" together.

The apparatus can be used to accurately locate the intersection of a body lumen with a branch vessel, thereby allowing fenestration of a graft at that location. It is to be noted that the first and second magnetic portions attract and couple across the fabric of the graft (ie. the fabric of the graft is between the coupled magnets). Generally, the second magnetic portion is located within the lumen of the graft and the first magnetic portion is located outside the graft.

The apparatus is suitable for in situ fenestration of a graft using only antegrade access. Once fenestration of the graft has been achieved, a stent or graft can be inserted into the branch vessel so that it forms a seal between the branch vessel and the main graft in the body lumen.

The term "fenestration" is defined herein as a hole or opening within a graft or prosthesis or to the process of producing such a hole or opening. The fenestration can have any shape including, but not limited to, rectangular, triangular or curvilinear (eg. circular, oval or the like). The term "in situ fenestration" refers to a hole or opening in a graft which is formed once the graft has been located within a patient's body, or to the process of forming that hole or opening whilst the graft is located within a patient's body.

The apparatus of the present invention is particularly useful to create a fenestration in a tubular graft. The term "tubular graft" as used herein refers to a tubular member suitable for placement into a body lumen, such as a duct or blood vessel, of a patient's body. The tubular member will have a proximal end and a distal end, with a lumen extending from the proximal end to the distal end.

The tubular graft can be formed from a biocompatible porous or nonporous material. Numerous grafts are available commercially or described in the art, but mention may be made of PTFE or ePTFE grafts, and knitted or woven grafts formed from suitable materials (eg. polyester, polyurethane or polypropylene). The graft can be tapered or can be of uniform cross-section. Optionally the graft can include a metallic stent located at the distal end, proximal end or both the distal and proximal ends in order to anchor the graft into position within the body lumen. Optionally, to avoid interference with the magnetic elements of the invention (ie. the first and second magnetic portions), the stent will be formed of a nonmagnetic material. The stent can be balloon expandable or self-expandable (eg. formed from shape memory materials, such as nitinol). Exemplary stents include wire mesh stents, helices, coils and other suitable configurations; see US 4,735,6645, US 6,635,080; US 6,203,568. In one embodiment the graft has anchoring stents at each end of a tubular member formed of ePTFE or knitted or woven material.

In one embodiment the catheter having the second magnetic portion further includes a cutting tool. The cutting tool is adapted to create a fenestration in the graft wall.

The cutting tool can be any suitable device capable of achieving fenestration in the graft. Selection of the cutting tool may vary depending upon the graft type and construction, and may be selected depending upon the position of the required opening. Suitable cutting tools include, but are not limited to, lasers, wires such as guide wires, ablation instruments, cutting tools (including catheters having such tools), needles and biopsy instruments.

Optionally more than one cutting tool can be used. For example, a sharp tipped guide wire could be used to create an initial fenestration which could then be expanded to the desired size using a balloon which has an inflated diameter of the required size. A standard percutaneous transluminal angioplasty (PTA) balloon, a vascular or cardiac coronary balloon or a percutaneous nephrostomy device could be used to enlarge the fenestration. The size of the fenestration can be further increased by deployment of a stent anchoring a supplementary graft in the branch vessel, or by a further larger diameter balloon.

Optionally, the cutting tool can be held in position following initial fenestration of the graft, allowing introduction of a secondary cutting tool.

In one embodiment the first magnetic portion of the guide wire comprises a C-shaped or U-shaped magnet.

The first magnetic portion can be part of a guide wire or can be part of a catheter introduced using a guide wire. Thus, a thin flexible guide wire can be used to locate a branch vessel and then used to direct a stiffer, less steerable catheter so that the first magnetic portion is accurately located.

In one embodiment the second magnetic portion comprises a C-shaped or U-shaped magnet.

In one embodiment, the method of the invention employs a graft which remains attached to its catheter after the sheath is retracted. A suitable graft of this type is the ANAGONDA® graft of Vascutek Ltd., UK (see US 6,635,080). Generally the catheter used for introduction of the graft will be separate from the catheter having the second magnet portion. However it could also be possible to design a combined catheter able to include both of these features.

In one embodiment, where the graft is to be inserted in a blood vessel, the method of the invention employs a graft which can be at least partially collapsed to allow blood flow between the outer surface of the graft and the inner lumen of the blood vessel until fenestration has been achieved. Such perfusion avoids the risk of ischaemia and consequent damage to tissue.

In one aspect, the present invention provides a method for location of a site for in situ fenestration of a tubular graft, said method comprising:
a) locating a guide wire in a branch vessel of a body lumen and providing a first magnetic portion in the branch vessel;
b) positioning the graft within the body lumen;
c) locating a catheter within the lumen of the graft, wherein said catheter has a second magnetic portion;
d) allowing the second magnetic portion of the catheter to be attracted to and dock with the first magnetic portion; and
e) using the docked second magnetic portion of the catheter to guide a cutting tool to a suitable site for fenestration of the graft.

In one embodiment, the present invention provides a method for in situ fenestration of a tubular graft, wherein a fenestration step (step f) is conducted after step e) by advancing a cutting tool (such as a guide wire with a sharp tip or needle tipped catheter) through the catheter in the graft lumen until it contacts and penetrates the fabric of the graft. This cutting tool can then be used to provide access to the branch vessel. An initial fenestration of the graft is achieved by the sharp tip of the cutting tool and the size of the fenestration can then be increased to the desired size by using a secondary cutting tool, for example by locating a deflated balloon along the cutting tool guide wire until the balloon partially crosses the fabric. The balloon can then be inflated so that the fenestration achieves the required size. Once enlargement of the fenestration has been accomplished, a supplementary (secondary) graft can be inserted through the fenestration into the branch vessel and deployed, so that it forms a seal between the branch vessel and the tubular graft. The secondary graft will normally be tubular and comprise stent(s) analogous to the main graft. The balloon size will be selected according to the stent type and size used in the supplementary graft. A balloon of smaller diameter (eg. 2 to 5 mm) can conveniently be used where the supplementary graft is to be anchored at the fenestration site using a balloon-expandable stent. A balloon of larger diameter (eg. 6 to 10 mm) can conveniently be used where the supplementary graft is to be anchored at the fenestration site using a self-expandable stent. The size of the fenestration to be achieved by the cutting tool will be pre-selected depending upon the patient's anatomy and choice of stent for the supplementary graft, and the appropriate cutting tool deployed.

In step a) the guide wire will normally be passed up the body lumen to the intersection with the branch vessel, and then into the branch vessel itself. Thus, all manipulations and procedures can be conducted from a single entry point in the patient, thereby simplifying the process.

If required, the method of the present invention can include a further step, step g), comprising insertion of a suitably sized graft within the branch vessel.

In one embodiment two or more fenestrations can be created in a single graft. Such an arrangement can be required where two or more branch vessels intersect with a single body lumen. Examples include the renal arteries at their intersection with the aorta. The method of the present invention can be adapted as required, and a tertiary graft can be inserted into the second branch vessel.

Thus in one embodiment the present invention provides a method for in situ fenestration of a tubular graft, said method comprising:
a)
   i) locating a first guide wire in a first branch vessel of a body lumen and providing a first magnetic portion in the first branch vessel;
   ii) locating a second guide wire in a second branch vessel of the body lumen and providing a third magnetic portion in the second branch vessel;
b) positioning the graft within the body lumen;
c) locating a catheter within the lumen of the graft, wherein said catheter has a second magnetic portion;
d) allowing the second magnetic portion of the catheter to be attracted to and dock with the first magnetic portion;
e) using the docked second magnetic portion of the catheter to guide a cutting tool to a suitable site for a first fenestration of the graft;
f) using said cutting tool to fenestrate the graft;
g) inserting a suitably sized graft within the branch vessel;
h) detaching the second magnetic portion of the catheter from the first magnetic portion;
i) allowing the second magnetic portion of the catheter to be attracted to and dock with the third magnetic portion; and
j) using the docked second magnetic portion of the catheter to guide a cutting tool to a suitable site for a second fenestration of the graft.

In one embodiment, step f) can be repeated to produce the second fenestration of the graft. Optionally, step g) can be repeated for the second branch vessel and the magnetic portions can then be detached.

In one embodiment, a high strength magnet, such as a magnet based on neodymium (eg. a neodymium iron boron magnet), is used in one, two or each magnetic portion. A "high strength" magnet is a magnet able to remain docked to the other magnetic portion as the cutting tool is passed through the fabric of the graft. Magnets based on other materials, such as samarium cobalt, could also be used. The magnets can be coated or injection moulded with other materials to optimise their suitability for *in vivo* use.

In one embodiment, the attractive magnetic force between the magnetic first or third magnetic portions with the second magnetic portion is greater than the force required to penetrate the graft fabric with the cutting tool.

In one embodiment, the magnetic attractive force of one, two or each magnetic portion is at least 1 N, preferably over 1.5N, more preferably over 1.9N.

In one embodiment, the magnetic field strength of each magnetic portion is at least 1000 A/m, preferably at least 1500 A/m, or even as high as 4000 A/m. There is no upper limit in the magnetic field strength suitable, which is limited only by the materials available and size of the magnetic portion possible.

The attractive magnetic force between the first or third magnetic portions with the second magnetic portion increases with the size of the magnets used. However, there is a limitation in size of each magnet imposed by the need to insert the magnetic elements of the apparatus into the blood vessels without causing damage to the vessels. Furthermore, an introducer may be used to minimise damage to the blood vessel lumen, which will impose a maximum cross-sectional diameter on the magnetic portions.

In one embodiment, a guide wire introduces a catheter which includes the first magnetic portion. Optionally, this catheter includes a snare which is designed to grasp and secure the cutting tool following initial penetration of the graft fabric.

The snare can be a manually operable device able to grasp the cutting tool once it has penetrated the graft fabric. The snare can be, for example, a loop of flexible wire, a clip, resilient catch, plate or other device able to releasably grasp the cutting tool and which can be operated by the user from the distal end of the guide catheter. Once the cutting tool is grasped by the snare, it is held in a locked configuration. Once the cutting tool is held in a locked configuration, accidental disengagement of the docked magnetic portions will have no effect on the ability to conduct subsequent steps, and a secondary cutting tool can be used to apply greater penetrative force to the initial fenestration site in order to expand the size of the fenestration. For example, the fenestration can be increased to the desired size by locating a deflated balloon at the fenestration site. The balloon can then be inflated to the required size of the fenestration. Once the desired size of fenestration has been achieved, the snare is disengaged, thus releasing the cutting tool from its locked configuration so that the cutting tool can be withdrawn.

Thus, in one embodiment, a guide wire is used to introduce a catheter bearing a first magnetic portion at its proximal end. The distal end of the catheter will protrude at least partially out from the patient's body. The proximal end of the catheter includes a snare which in this embodiment is a loop of flexible wire. The diameter of the annulus described by the loop of flexible wire can be manipulated by the catheter operator, for example, the annulus of the loop can be reduced by pulling at the end of the flexible wire at the distal end of the catheter.

In this embodiment, the cutting tool comprises a notch or indentation configured to receive the loop of flexible wire. Once the loop of wire is in position, and the cutting tool has penetrated the fabric of the graft, the annulus of the loop is reduced so that the loop tightens around the notch or indentation of the cutting tool. The loop of flexible wire is then held within the notch or indentation so that the cutting tool is held in a locked configuration.

In one embodiment, the loop of flexible wire is situated on the outside of the catheter.

In another embodiment, the loop of flexible wire is situated within the lumen of the catheter and is advanced through an aperture in the catheter.

In one embodiment, the snare is a plate having an aperture through which the cutting tool is passed. In this embodiment, the plate is brought into contact with the cutting tool by movement of the plate itself or by a wire or thread extending from the plate which can be tightened by the operator at the distal end of the guide catheter. Tightening of the wire or thread increases the contact of the plate against the cutting tool, thereby holding the cutting tool in position, in a locked configuration.

Thus in one embodiment, the present invention provides a method for in situ fenestration of a tubular graft, which method comprises:
a)
   i) locating a guide wire in a branch vessel of a body lumen;
   ii) locating a catheter in said vessel, said catheter having a first magnetic portion and a snare configured to releasably engage a cutting tool;
b) positioning the graft within the body lumen;
c) locating a catheter within the lumen of the graft, wherein said catheter has a second magnetic portion;
d) allowing the second magnetic portion of the catheter to be attracted to and dock with the first magnetic portion;
e) using the docked second magnetic portion of the catheter to guide a first cutting tool to a suitable site for fenestration of the graft;
f)
   i) using said first cutting tool to fenestrate the graft; and
   ii) releasably engaging the first cutting tool with the snare.

The snare can be a loop of flexible wire or plate as described above, or could be a resilient clip, or the like.

Once each fenestration has been achieved, a supplementary (secondary) graft can be inserted through the fenestration into each branch vessel and deployed, so that it forms a seal between the branch vessel and the tubular graft. The supplementary graft will normally be tubular and comprise stent(s) analogous to the main graft.

Fenestration of fabric materials is currently achieved using a combination of wires, needles and balloons, and is achieved by essentially "tearing" the fabric at the required location to achieve a fenestration of the required size. However the presence of torn yarn will affect graft stability adversely. Thus McWilliams et al., (J. Endovasc Ther (2204) 11:170-174) express concerns regarding the long-term stability of the fabric tears, and Tse et al., (J. Endovasc Ther (2007) 14:158-167) notes torn yarn in the graft at the fenestration site and expresses concern over continuous enlargement of fabric holes with time.

There is thus a need for improved graft fabric adapted for fenestration, and in particular to a fabric able to resist unwanted propagation of tears.

The present invention thus provides a fabric graft wherein an area of the graft is reinforced by a mesh.

The graft fabric could be any conventional graft fabric suitable for endovascular use, for example could be woven or knitted polyester, polyurethane or polypropylene. The graft fabric could also be PTFE or ePTFE.

In one embodiment, the graft fabric has a cross-sectional thickness of less than 0.15 mm.

In one embodiment, the graft fabric has a water permeability of less than 500 mL/cm²/min, preferably less than 250 mL/cm²/min.

In one embodiment the mesh is formed by an arrangement of strands. The strands can be made from wire (for example stainless steel), from polymeric material or from multi-filament or monofilament yarn. Suitable polymeric materials and yarn materials include polyester, PTFE, polypropylene, polyethylene and the like. High tenacity orientated polypropylene (such as Dyneema^{®}) may be particularly suitable due to its high strength/weight ratio.

The mesh can be attached to the fabric by any suitable means and mention can be made of sewing, adhesive bonding, thermal bonding and the like. Alternatively the strands forming the mesh can be woven or knitted into the fabric itself during construction.

The mesh will comprise a pattern of open areas between the strands. The open areas can be regular shapes, for example can be square, rectangular or hexagonal. An hexagonal (honeycomb) pattern is preferred.

The area of the fabric graft reinforced by mesh is preferably the area to be subjected to fenestration, particularly fenestration in situ. During fenestration, the edges of the tear will be limited to an open area of the mesh. Fraying of the graft beyond this defined open area will therefore be limited. The size of the open area in the mesh will be selected to be suitable for fenestration.

The present invention will now be further described having reference to the following, non-limiting figures in which:
Figure 1 is a schematic cross-section of an aneurismal aorta showing the renal arteries and first and second magnetic tipped guide wires located therein;
Figure 2 is the aneurismal aorta of Figure 1 showing a graft located therein and crossing the intersection of the renal arteries;
Figure 3 shows the aneurismal aorta of Figure 2 in which a magnetic tipped catheter has been inserted into the lumen of the graft;
Figure 4 shows detail of the docking of the magnetic tipped catheter with a first magnetic tipped guide wire;
Figure 5 shows the aneurismal aorta of Figure 3 showing the docking of the magnetic tipped catheter with the magnetic tipped guide wire;
Figure 6 shows detail of the magnetically docked catheter and guide wire guiding a cutting instrument as it penetrates the fabric of the graft;
Figure 7 shows detail of a balloon catheter being passed over the guide wire in its unexpanded state;
Figure 8 shows the balloon catheter of Figure 7 in inflated form;
Figure 9 shows the aneurismal aorta of Figure 5 having a graft deployed in a branch vessel;
Figure 10 shows detail of the branch vessel graft at the intersection with the main graft;
Figure 11 is the aneurismal aorta of Figure 9 showing the graft fully deployed and including grafts in each of the renal arteries;
Figure 12 shows detail of an embodiment of the invention in which the cutting tool has been advanced through the fabric of the graft, and is in place to be secured by a snare; and
Figure 13 shows the snare in use, with the cutting tool held in its locked configuration.

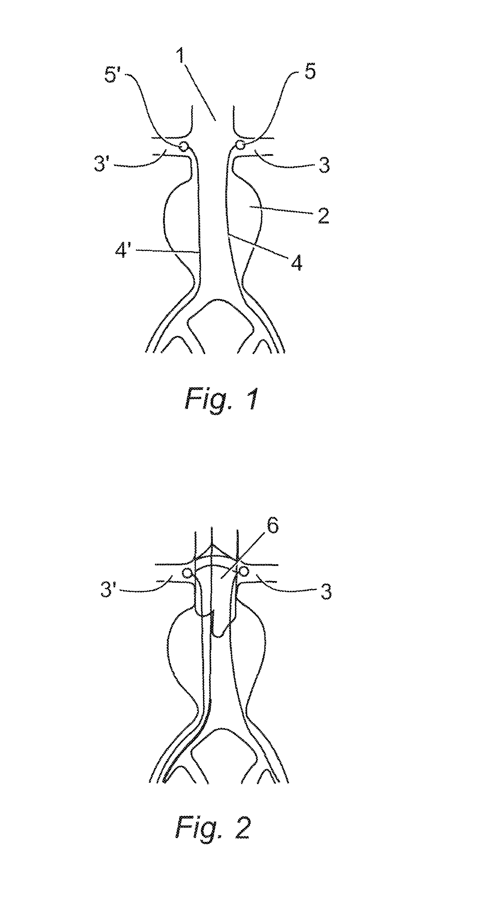

Figure 1 depicts a cross section of the aorta (1) showing a significant aneurysm (2) below the renal arteries (3, 3'). A guide wire (4) has been inserted into the left renal artery (3) and bears a magnetic portion (5) (corresponding to the third magnetic portion) at its tip. As depicted, a second guide wire (4') has been inserted into the right renal artery (3') and also bears a magnetic portion (5') (corresponding to the first magnetic portion) at its tip. Once the guide wires (4, 4') are correctly located a main graft (6) is inserted into the lumen of the aorta (1) (see Figure 2). Since the aneurysm is close to the intersection of the renal arteries the graft (6) covers the renal artery intersections and without fenestration would occlude blood flow into those branch vessels.

As clearly shown in Figure 2 the guide wires (4, 4') lie between the exterior of the graft (6) and the interior wall of the aorta (1). Once the graft has been inserted a catheter (7) is introduced into the aorta (1) and into the lumen of graft (6) and advanced to approximately the location of the intersection with branch vessels (3, 3'). Catheter (7) bears a magnetic portion (8) at its tip. Magnetic portion (8) corresponds to the second magnetic portion. A suitable catheter is the Terumo Destination 7F Introducer modified to include a magnetic ring at its tip. This sheath can be used to introduce all the other components. The magnetic portion (8) of catheter (7) has been selected to be of opposite polarity to magnetic portions (5) and (5') of the guide wires (4, 4'). Thus, if the magnetic portion (8) is of north polarity, the magnetic portions (5, 5') will be of south polarity, and vice versa. Once the magnetic tip (8) of catheter (7) is at the approximate location of the intersection with branch vessels (3, 3') it is manipulated until it is attracted to and docks with magnetic portion (5') of the right renal artery (3'). Of course, it is possible for the left renal artery (3) to be processed first and in this situation the magnetic portion (8) would be manipulated until it is attracted to and docks with magnetic tip (5) located in the left renal artery (3).

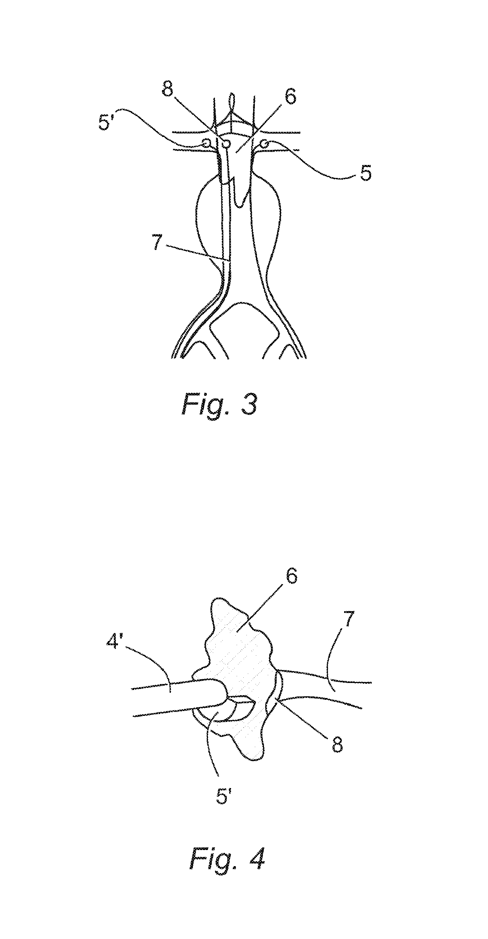

As is more clearly shown in Figure 4 the magnetic portion (5') of guide wire (4') is docked with the magnetic portion (8) of catheter (7), with the fabric wall of the graft (6) therebetween. The strength of the magnets (5, 5') and (8) is selected to be strong enough to avoid unintentional release, whilst allowing intended release upon withdrawal of the guide wire (4) or (4') or of the catheter (7). For example, the magnetic material can be based on neodymium. As illustrated, the magnetic portion (5, 5') is approximately C-shaped whilst magnetic portion (8) is formed as a ring. Other configurations are also possible.

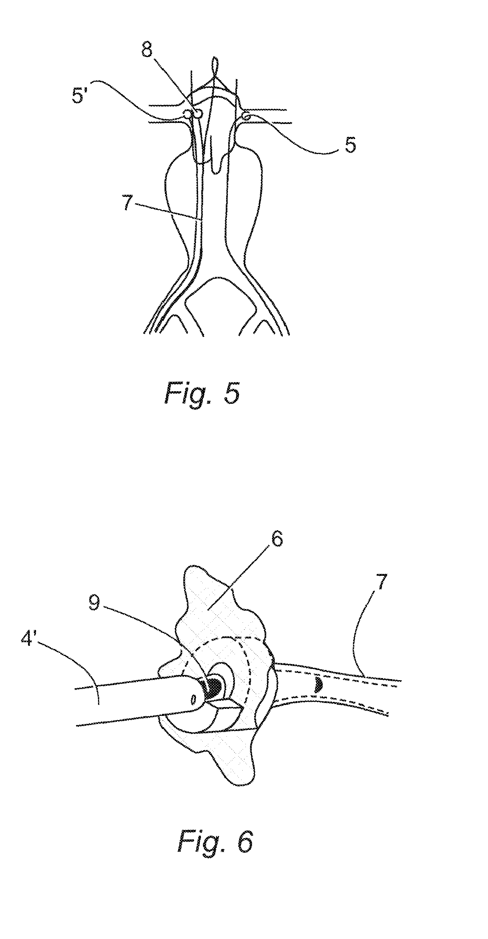

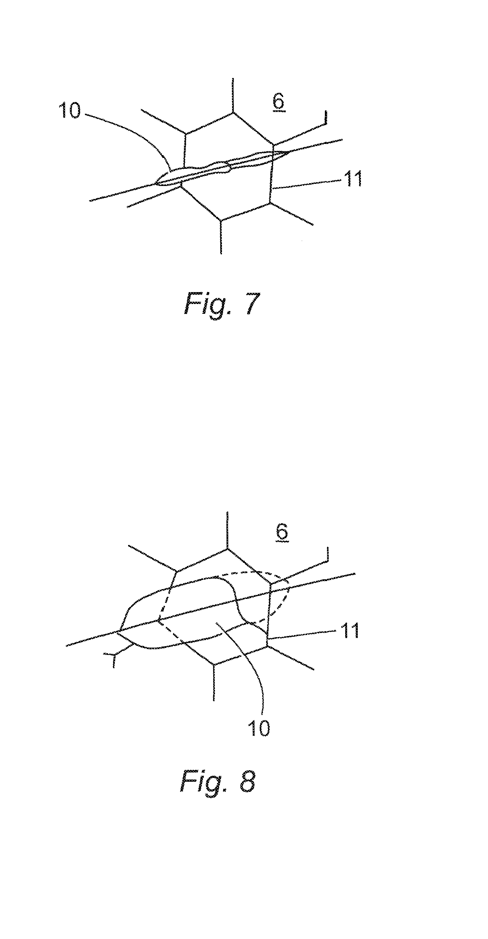

Once the magnetic portions (5' and 8) have securely docked (see Figure 5), a cutting tool (9) is inserted through the catheter (7) until it contacts and then penetrates the fabric of graft (6). As illustrated in Figure 6 the cutting tool (9) is a sharp tipped guide wire. A suitable cutting tool (9) is an Optitorque 4F angiocatheter with a 18G needle on top. Once the initial penetration of the graft (6) has been achieved using the cutting tool (9), the size of the fenestration can be expanded using a second cutting tool, which in the illustration is a balloon catheter (10). An expanded detail of the wall of graft (6) is illustrated in Figure 7 and depicts the deflated balloon (10) advanced to a point where the wall of graft (6) is about half way down the length of the balloon. Once the balloon (10) is located approximately midway across the wall of graft (6) the balloon (10) is expanded by inflation. The pressure of the inflated balloon (10) increases the size of the fenestration. The inflated diameter of balloon (10) will be selected to achieve the fenestration size required. A 3 - 4 mm diameter PTCA balloon is suitable for applications where the secondary graft will deploy a 7 mm diameter balloon expandable stent. After fenestration has been completed the balloon catheter (10) can be deflated and withdrawn.

As illustrated in Figures 7 and 8 the fabric of the graft (6) is reinforced by a mesh (11). Mesh (11) is shown formed from strands arranged in a hexagonal pattern and the fenestration is located within one hexagonal open area of the mesh. Subsequent tearing or elongation of the fenestration is limited by the strands which serve to reinforce the graft fabric and which bound the hexagonal open area. A secondary graft (12) is then passed through the fenestration and deployed in the right renal artery (3'). A Veripath 7F guiding catheter can be used for deployment of the secondary graft and its stent. The process of Figures 3 to 8 is then repeated for the left renal artery (3).

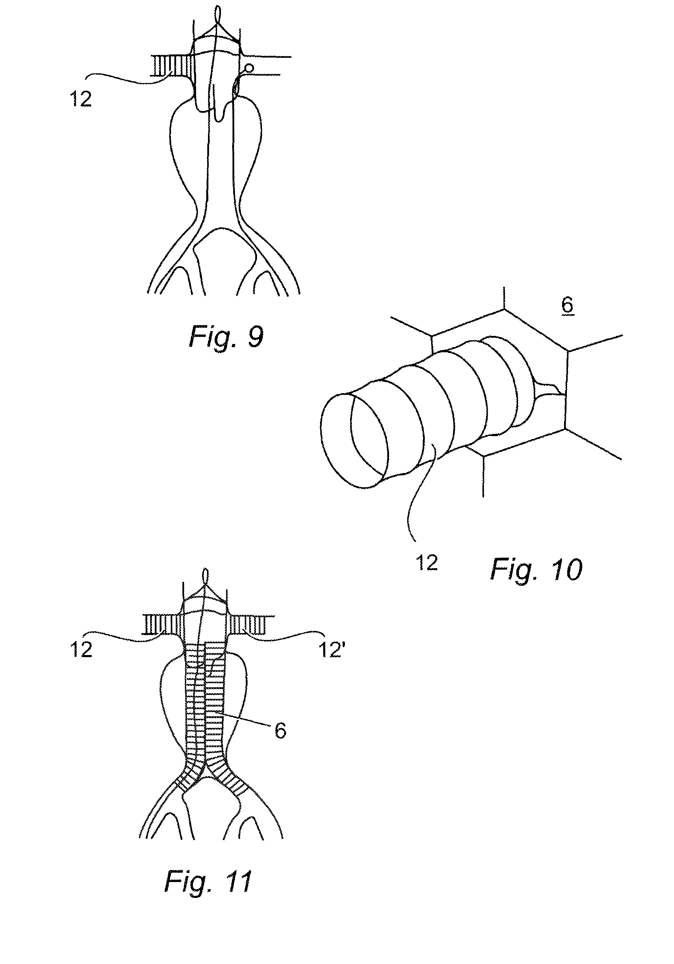

Figure 10 shows the detail of a deployed secondary graft (12) at its junction with the main graft (6).

Figure 11 shows the fully deployed graft in which the main graft (6) is connected to secondary grafts (12 and 12') in the right and left renal arteries respectively.

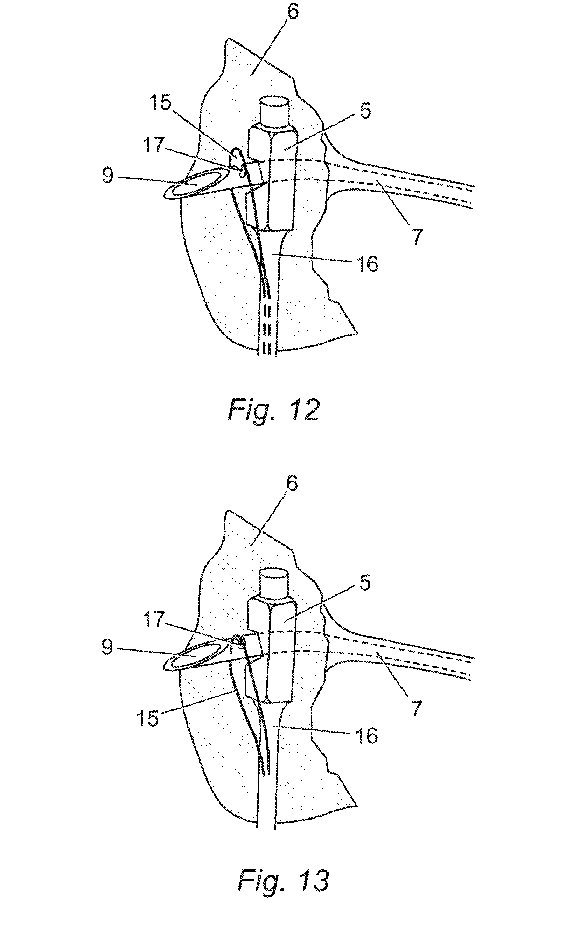

Figures 12 and 13 show a further embodiment of the invention in use. In this embodiment, the magnetic portion (5) forms part of catheter (16) which is advanced up a guide wire pre-located in the branch vessel. The catheter (16), graft (6), and catheter (7) bearing the second magnetic portion (8) and including a first cutting tool (9) are placed within the patient as described above. Once the magnetic portions (5, 8) have securely docked, the cutting tool (9) is advanced along the catheter (7) to magnetic portion (8, not shown) until it contacts and then penetrates the fabric of graft (6). As illustrated in Figure 12, the cutting tool (9) is a sharp tipped wire. A suitable cutting tool is an Optitorque 4F angiocatheter with a 18G needle on top.

Once the initial penetration of the graft (6) has been achieved using the cutting tool (9), the cutting tool (9) is advanced through the fabric of the graft (6) a small distance, until the cutting tool passes through snare (15) of the catheter (16). As illustrated, snare (15) comprises a loop of flexible wire and the cutting tool (15) passes through the annulus of the loop. The diameter of the loop of wire in snare (15) (its annulus) can be altered by the catheter operator by manipulation at the distal end of the catheter. Cutting tool (9) includes a notch or indentation (17), near its tip and configured to engage with snare (15). When the cutting tool (9) is in position, snare (15) is engaged.

Engagement of snare (15) is achieved by reduction of the diameter of the wire loop of snare (15) so that the loop tightens around the cutting tool (9) and becomes tightly seated within the notch (17) causing the cutting tool (9) to be in a locked configuration. Figure 13 shows the embodiment as described above, with the cutting tool (9) in its locked configuration. The snare (15) is shown engaging the cutting tool (9), secured by the notch (17).

Once the cutting tool (9) is in its locked configuration as described above, a deflated balloon can be advanced until it is positioned partially through the initial fenestration in graft (6) produced by cutting tool (9). The size of the fenestration can then be expanded by inflating the balloon as described above in Figures 7 to 8. Disengagement of the docked magnetic portions (5, 8) will not now affect the subsequent process. Once the balloon has been deployed and the size of fenestration achieved is suitable for allowing passage of the secondary graft (and stent if required), the balloon is deflated and withdrawn. The cutting tool (9) can be released from its locked configuration by increasing the diameter of the wire loop of the snare (15). The cutting tool (9) can be withdrawn before or after insertion and deployment of the secondary graft, as required.

Whilst the apparatus and method of the present invention is illustrated above with reference to the intersection of the renal arteries with the aorta, the present invention is not so limited and covers other intersections of branch vessels with a body lumen, including single branch sites. The preferred main graft, as illustrated, is the ANACONDA^{®} stent graft of Vascutek Terumo, but again, the present invention is not limited to this graft.

The present invention will be further described with reference to the following non-limiting example.

### EXAMPLE 1: Penetration force of hypodermic needles through polyester fabric

This example describes the method used to determine the force required to push a hypodermic needle through a woven polyester endovascular fabric. The fabric had a water permeability of less than 500 mL/cm²/min and was less than 0.15 mm in cross-section. This report also includes the results achieved with a variety of different sized needles ranging from 16 - 25 gauge.

### Method

The method is identical to that specified in ISO 7198 - Cardiovascular Implants - Tubular Vascular Prostheses, Section 8.3.3.2 - *Determination of probe burst strength*, except that hypodermic needles of differing sizes are used to penetrate the fabric (instead of the hemispherical probe described in ISO 7198).

All testing was carried out using woven endovascular polyester fabric with the woven batch number wme22002ev.

As a comparison, the magnetic attraction force between two cylindrical magnets through the fabric was also measured. In this testing two magnet sizes were used; 1) 2.5 diameter, 3 mm length and 2) 2.0 diameter 3 mm length.

Needles of gauge 16 (nominal outer diameter = 1.65 mm), 19 (nominal outer diameter = 1.07 mm), 22 (nominal outer diameter = 0.71 mm) and 25 (nominal outer diameter = 0.51 mm) were used.

### Results

**Table 1 - Penetration/Attraction force results**

| | Penetration Force (Newtons) | | | | Magnetic Attraction Force (Newtons) | |
|---|---|---|---|---|---|---|
| | 16 Gauge | 19 Gauge | 22 Gauge | 25 Gauge | 2.5 x 3 mm magnet | 2.0 x 3 mm magnet |
| Mean | 0.7525 | 0.4333 | 0.2341 | 0.1796 | 2.38 | 1.91 |

As shown in Table 1, the force increases as the needle becomes larger (a lower number for the needle gauge corresponds to a larger diameter). The results in Table 1 show that the attraction force between the two magnets through the fabric is significantly higher than the penetration force of even the largest needle.

As can be seen from Table 1 it is possible to achieve magnetic attachment forces higher than the penetration force using needles of size up to and including 16 gauge.

As noted above, the present invention also relates to:
an apparatus for locating a site for in situ fenestration of a tubular graft, said apparatus comprising
   i) a first magnetic portion, and
   ii) a catheter having a second magnetic portion, wherein the first magnetic portion and the second magnetic portion are attracted to each other.

The catheter may comprise one or more cutting means selected from lasers, wires, balloons, ablation instruments, needles and biopsy instruments.

Optionally, the first magnetic portion comprises a C-shaped or U-shaped magnet, and/or the second magnetic portion comprises a C-shaped or U-shaped magnet.

The said apparatus may further comprise a tubular graft for delivery into a body lumen, wherein the graft remains attached to its catheter after the sheath is retracted, and optionally, where the body lumen is a blood vessel the graft can be at least partially collapsed to allow blood flow between the outer surface of the graft and the inner lumen of the blood vessel until fenestration has been achieved.

Optionally the first magnetic portion is located on a catheter, wherein optionally the catheter having the first magnetic portion further comprises a snare, and optionally the snare comprises a loop of flexible wire, configured to extend from the distal end of the catheter, and optionally the cutting tool comprises at least one notch or indentation.

The present invention also relates to a method for location of a site for in situ fenestration of a tubular graft is also provided, said method comprising:
a) locating a guide wire in a branch vessel of a body lumen and providing a first magnetic portion in the branch vessel;
b) positioning the graft within the body lumen;
c) locating a catheter within the lumen of the graft, wherein said catheter has a second magnetic portion;
d) allowing the second magnetic portion of the catheter to be attracted to and dock with the first magnetic portion of said guide wire; and
e) using the docked second magnetic portion of the catheter as a positioning means for fenestration of the graft.

Additionally, a method for in situ fenestration of a tubular graft is also provided, said method comprising:
a) locating a guide wire in a branch vessel of a body lumen and providing a first magnetic portion in the branch vessel;
b) positioning the graft within the body lumen;
c) locating a catheter within the lumen of the graft, wherein said catheter has a second magnetic portion;
d) allowing the second magnetic portion of the catheter to be attracted to and dock with the first magnetic portion of said guide wire;
e) using the docked second magnetic portion of the catheter as a positioning means for fenestration of the graft; and
f) advancing a cutting tool until it contacts and penetrates the fabric of the graft.

Optionally, said cutting tool is a guide wire or needle tipped catheter, and optionally the cutting tool further comprises a balloon catheter. Optionally step f) further comprises advancing the balloon until it partially crosses the fabric and then inflating the balloon to achieve the required size of the fenestration.

In some embodiments of any said method above, a secondary graft is inserted through the fenestration into the branch vessel, and optionally, the secondary graft is tubular and comprises one or more stents.

A method for in situ fenestration of a tubular graft is also provided, said method comprising:
a)
   i) locating a first guide wire in a first branch vessel of a body lumen and providing a first magnetic portion in the first branch vessel;
   ii) locating a second guide wire in a second branch vessel of the body lumen and providing a third magnetic portion in the second branch vessel;
b) positioning the graft within the body lumen;
c) locating a catheter within the lumen of the graft, wherein said catheter has a second magnetic portion;
d) allowing the second magnetic portion of the catheter to be attracted to and dock with the first magnetic portion;
e) using the docked second magnetic portion of the catheter to guide a cutting tool to a suitable site for a first fenestration of the graft;
f) using said cutting tool to fenestrate the graft;
g) inserting a suitably sized graft within the branch vessel;
h) detaching the second magnetic portion of the catheter from the first magnetic portion;
i) allowing the second magnetic portion of the catheter to be attracted to and dock with the third magnetic portion; and
j) using the docked second magnetic portion of the catheter to guide a cutting tool to a suitable site for a second fenestration of the graft.

Optionally in this method, a secondary graft is inserted through the first fenestration into the first branch vessel and a tertiary graft is inserted through the second fenestration into the second branch vessel. Optionally the secondary and tertiary grafts are each tubular and each comprise one or more stents.

The present application also provides a method for in situ fenestration of a tubular graft, said method comprising:
a)
   i) locating a guide wire in a branch vessel of a body lumen;
   ii) locating a catheter in said vessel, said catheter having a first magnetic portion and a snare configured to releasably engage a cutting tool;
b) positioning the graft within the body lumen;
c) locating a catheter within the lumen of the graft, wherein said catheter has a second magnetic portion;
d) allowing the second magnetic portion of the catheter to be attracted to and dock with the first magnetic portion;
e) using the docked second magnetic portion of the catheter to guide a first cutting tool to a suitable site for fenestration of the graft;
f)
   i) using said first cutting tool to fenestrate the graft; and
   ii) releasably engaging the first cutting tool following fenestration by means of the snare.

Optionally, in this method,the cutting tool further comprises a balloon catheter, and optionally the method further comprises advancing the balloon until it partially crosses the fabric of the graft and inflating the balloon to achieve the required size of the fenestration. Optionally a secondary graft is inserted through the fenestration into the branch vessel and deployed. Also optionally the secondary graft is tubular and comprises one or more stents.

## Claims

1. A graft fabric adapted for fenestration, wherein an area of the graft is reinforced by a mesh comprising an arrangement of strands, **characterised in that** the strands forming the mesh are woven or knitted into the fabric itself during construction.

2. The fabric as claimed in claim 1 wherein the mesh comprises wire, polymeric material or multi-filament or monofilament yarn.

3. The fabric as claimed in claim 2 wherein the mesh is polyester, PTFE, polypropylene, or polyethylene.

4. The fabric as claimed in claim 2 wherein the mesh comprises stainless steel.

5. The fabric as claimed in any of claims 1 to 4 wherein the fabric has a cross-sectional thickness of less than 0.15mm.

6. The fabric of any one of claims 1 to 5 wherein the fabric has a water permeability of less than 500mL/cm²/min.

7. The fabric as claimed in claim 6 wherein said graft fabric has a water permeability of less than 250mL/cm²/min.

8. The fabric as claimed in any one of claims 1 to 7 wherein the mesh comprises a pattern of open areas between said strands.

9. The fabric of claim 8 wherein the open areas are regular shapes, optionally selected from square, rectangular or hexagonal.

10. The fabric as claimed in claim 9 wherein said open areas are formed in a hexagonal pattern.

11. The fabric as claimed in any one of claims 1 to 10 wherein the area of said fabric reinforced by the mesh is the area to be subject to fenestration.

12. The fabric as claimed in any one of claims 1 to 11, wherein the fabric is PTFE, ePTFE or is woven or knitted polyester, polyurethane or polypropylene.

13. A graft comprising a fabric as claim in any one of claims 1 to 12.
